# EUROPEAN PATENT SPECIFICATION

(11) **EP 0 481 396 B1**
(45) Date of publication and mention of the grant of the patent: **11.01.1995**
(21) Application number: 91117472.0
(22) Date of filing: 14.10.1991
(51) Int. Cl.: A61K 35/78, A61K 31/70

(54) **Tannin extract of sweet chestnut wood, a process for the preparation thereof and its use**
Tanninextrakt von Kastanienholz, Verfahren zu deren Herstellung und deren Verwendung
Extrait de tannin de bois de châtaignier, son procédé de préparation et son utilisation

(30) Priority: 15.10.1990 YU 1933/90
(43) Date of publication of application: 22.04.1992
(73) Proprietor: TANIN SEVNICA KEMICNA INDUSTRIJA p.o., SI-68290 Sevnica (SI)
(72) Inventor: Mirosevic, Roko, YU-68290 Sevnica, Slovenia (YU)
(74) Representative: von Füner, Alexander, Dr.

(56) References cited:
- DE-A- 1 492 909
- H. WAGNER 'Pharmazeutische Biologie - Drogen und ihre Inhaltsstoffe' 1980 ,GUSTAV FISCHER VERLAG , STUTTGART page 228-236
- LIEGIGS ANN. CHEM. vol. 721, 1969, pages 186 - 193; W. MAYER ET AL.: 'Über die Gerbstoffe aus dem Holz der Edelkastanie und der Eiche, IV: Die Struktur des Castalgins'

## Description

The present invention belongs to the field of chemistry and relates to a tannin extract of sweet chestnut wood as a novel, i.e. hitherto not manufactured product having novel characteristics, especially as a natural agent for the prevention of diarrhoea in animals, as well as to a process for the preparation thereof and to its use.

In animal husbandry, especially in intensive animal breeding, there are several gastrointestinal diseases causing many losses in young animals.

Current medical and economic problems in intensive animal breeding should be solved by means of agents, which protect from a disease in a safe and natural way.

There existed the need for a natural preparation for preventing diarrhoea in animals, which preparation should be organoleptically acceptable and would not affect the meat quality of said animals.

As agents against diarrhoeas in animals, antibiotics and chemotherapeutica are widely used. The use of antibiotics and chemotherapeutica represents, however, a big problem due to various side effects, such as residues in meat, resistance, waiting period before slaughter, toxic effects in long-term treatment and allergic reactions.

A similar known process is the preparation of tannin for tanning, i.e. technical tannin, which process takes place in the same device as the inventive process. For preparing tannin for tanning, however, various kinds of wood may be used, also with bark, wood particle size is not very important, and process parameters for the known and novel processes differ, which is understandable since pharmaceutical criteria need not be considered in a known process.

In DE-A-1492909 there are disclosed preparations for promoting animal growth, which preparations contain, in addition to conventional fodder components (there was no sugar), a small portion of commercially available (cf. page 5, 1st paragraph) tanning extract. In Pharm. Biolcgie - Drogen und ihre Inhaltsstoffe G.Fischer Verlag, page 228-236,there is given a very general review of tanning substances and characteristics thereof.

The inventive tannin extract of sweet chestnut wood comprises a pulverized solution of a) a thickened aqueous extract of sweet chestnut wood (*Castanea Sativa Mill.*) without bark and sap-wood, said wood having an age from 40 to 50 years, and b) microbiologically acceptable sugar, the ratio dry substance of extract a)/sugar being from 1:2 to 1:6.

The age of wood is very important because the content and the quality of tannin as the active substance of the extract depend upon said age.

As sugar the common sugar, i.e. sucrose, is used, which must, however, correspond to pharmacopoeia regulations.

The pulverization is carried out e.g. by spray drying.

A process for preparing tannin extract of sweet chestnut wood for veterinary use is carried out in such a way that sound wood of sweet chestnut, of an age of 40 to 50 years, without bark and sap-wood, in the form of logs, which is ground into particles having a length of 6 to 10 mm, a thickness of 0.6 to 1.2 mm and a width of 4 to 8 mm, is extracted with water, the extraction solution is thickened to the dry substance content of about 50%, to the liquid extract a solution of microbiologically acceptable sugar is added in order to achieve a ratio dry substance of extract/sugar 1:2 to 1:6, it is stirred to complete homogeneity and pulverized.

Sweet chestnut wood is used, which must be absolutely sound, neither friable nor cancerous.

The wood should be ground since only in this way it is made available for the solvent in the extraction phase.

The addition of the sugar solution is the most essential feature since on the basis of this solution novel products with novel characteristics and novel use range are obtained.

Due to technological reasons - removal of water and stirring - an about 50% sugar solution is the most convenient one.

It should be understood that the equipment for the technological process should be made of a material which is inert for substances to be treated (e.g. thickening vats made of Cr/Ni). Said equipment should be adequately purified before use since a veterinary grade cannot be achieved otherwise.

The novel product obtained according to the invention is used either in the form of an appropriate veterinary preparation or as a fodder additive for the prevention of diarrhoeas in animals. It is obtained from natural materials and does not necessitate any waiting period before slaughter, i.e. animal meat and milk are suitable for human use without reservations. The product acts as adstringent - it forms a thin layer of unsoluble proteins on the surface of the mucus and thus prevents water transfer. It coagulates blood proteins and thus reduces bleedings. As mentioned before, the antibiotics and chemotherapeutica have a deep action and hence affect the meat quality, whereas the novel product of the invention only exhibits a surface action.

In comparison with pure tannin (*Acidum tannicum*), the product of the invention has a reduced activity due to the content of natural sugar, which results in a better compatibility and easier secretion under the same effect being made possible.

Ready-to-use preparation contains, in addition to the extract of the invention, conventional veterinarily acceptable additives (carriers, adjuvants).

In more serious cases the novel product is used in combination with chemotherapeutica or antibiotics.

Animals to be treated are mammals, such as pigs, adult cattle, calves, dogs, cats, rabbits, and also poultry (hens).

The preparation is used especially for the therapy of alimentary dysentery in weaned piglets and calves, of dysentery in fattening pigs before transport, of winter dysenteries in cattle and of other gastrointestinal disorders accompanied by diarrhoea.

The art of dosage depends upon the animal kind, body weight, age of the subject to be treated, its state of health etc. The preparation is administered as a preventive measure as well as in curative treatment in admixture with food or dissolved in water, always p.o. The general optimum daily dose is about 0.2 g/kg of body weight of the animal. E.g. pigs are administered from 3 to 5 kg/t of food daily for 7 to 21 days; adult cattle daily from 2 to 8 big spoons of a solution containing a daily dose dissolved in 2 to 3 l water for 3 to 4 days; calves daily from 1 to 2 big spoons of the same solution for 3 to 4 days; poultry daily from 0.02 to 0.1 g for 5 days or 50-70 g/100 l water.

The invention is illustrated by the following Examples and pharmacological tests.

### Example 1

Absolutely sound sweet chestnut wood, of an age of 40 to 50 years, in the form of logs without bark and sap-wood, was ground into particles having the following dimensions:
- length of 6 to 10 mm
- thickness of 0.6 to 1.2 mm
- width of 4 to 8 mm
The extraction was carried out in eight autoclaves in countercurrent. In the extractor there was a pressure of 0.6 bar and a temperature of 110 °C. Pure hot water was led to the raw material. Water had to be soft with a pH = 2.9 - 3.2. It was heated in a pre-heater to 110 °C. For one extraction 17,000 l of hot water per 11.5 m³ of ground wood were needed. The solution obtained at extraction was thickened in an evaporator series to a concentration of 50% of dry substance (density 1.255 g/ml). The thickening took place in three bodies of 105 m² of heating surface each. Before thickening the evaporators had to be completely empty and cleaned.

Thickening conditions:

| | |
|---|---|
| - 1st body: | 96 °C, underpressure 0.1 bar |
| - 2nd body: | 85 °C, underpressure 0.4 bar |
| - 3rd body: | 56 °C, underpressure 0.9 bar |

The thickened solution (liquid extract) was pumped in a cleaned, steamed, covered Cr-Ni vat of 18 m³ volume. The vat was filled with 11 m³ of a thickened liquid extract with a density of 1.255 g/ml and then 4 m³ of a 50% sugar solution were added. It was heated to 50 °C and stirred for 3 hours, whereafter it was spray-dried.

The sugar solution was obtained by dissolving 2500 kg of microbiologically acceptable sugar in 2500 l of water heated to 50 °C, in a Cr-Ni vat, previously cleaned and steamed.

Spray-drying took place in a spray drier under the following conditions:

| | |
|---|---|
| - the temperature of the liquid extract | 47° to 48°C |
| - the temperature of the input air | 185°C |
| - the temperature of the output air | 75° to 76°C |

The final product, tannin extract of sweet chestnut wood, was filled in four-layer plastic coated sacs of 20 kg each. Storage should take place in a dry place.

### Example 2

### A veterinary composition - fodder additive

The final product obtained according to Example 1 is directly suitable to be used as a fodder additive.

### A solution

A daily dose (about 0.2 g/kg of body weight) of the product of the invention prepared according to Example 1 was dissolved in 2 to 3 l of water.

### PHARMACOLOGICAL TESTS

### 1.Acute toxicity

The test results showed very low toxicities of tannic acid and tannin extracts, resp. It was found that LD 1 for the product of the invention when administered p.o. to rats was 3000 mg/kg of body weight, LD 50 for the 76-78% tannin extract when administered p.o. to rats was 1333 mg/kg of body weight and LD 100 for tannic acid when administered p.o. to mice was 6000 mg/kg of body weight.

### 2.Subacute toxicity

A test was carried out in mice in a control group (without application of the product of the invention) and in a test group (10 animals in each group), which animals were administered p.o. 120 mg/kg/day of the product of the invention for 20 days. Body weight of mice at the end of the test was 25.7 g/mouse in the test group and 25.8 g/mouse in the control group. The haematological picture of mice is evident from the Table 1 below:

**TABLE 1**

| Haematological picture of mice | | | | |
|---|---|---|---|---|
| | erythrocytes x 10/l | haemoglobin g/l | haematocrit l | leukocytes x 10/l |
| A | 7.16 ± 0.71 | 139.8 ± 16.4 | 0.45 ± 0.044 | 6.27 ± 0.84 |
| B | 6.53 ± 0.46 | 129.1 ± 20.6 | 0.43 ± 0.049 | 6.55 ± 0.26 |
| A = test group B = control group | | | | |

The results show that the values of erythrocytes, haemoglobin and haematocrit are a little higher in the test group than in the control group.

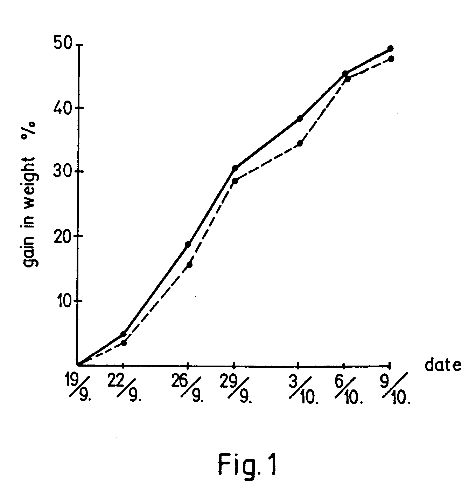

The second test was carried out in rats in a control group (without application of the product of the invention) and in a test group (6 animals in each group), which animals were administered p.o. a 1% solution of the product of the invention for 21 days. The weight increase in rats is shown graphically in Fig. 1, where the full line represents the test group and the dotted line represents the control.

The haematological picture of rats is evident from the Table 2 below:

**TABLE 2**

| Haematological picture of rats | | | | |
|---|---|---|---|---|
| | erythrocytes x 10/l | haemoglobin g/l | haematocrit l | leukocytes x 10/l |
| A | 7.07 ± 0.94 | 15.5 ± 1.4 | 46.5 ± 7.9 | 6.22 ± 2.71 |
| B | 6.79 ± 0.56 | 14.6 ± 1.8 | 43.7 ± 3.1 | 5.84 ± 1.37 |
| A = test group B = control group | | | | |

The results show that the values of erythrocytes, haemoglobin and haemocrit are a little higher in the test group than in the control group, which means that the blood picture in the test group has improved a little.

On the basis of the above results it can be stated that the product of the invention is practically non-toxic in the doses used.

### 3.Activity of the product of the invention

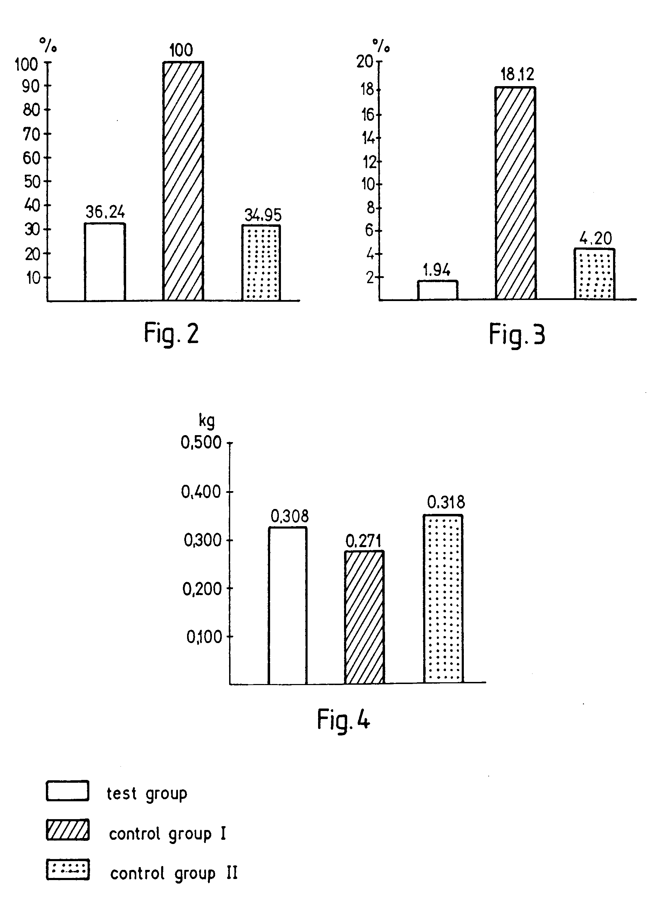

a) Activity in prevention of gastroenteritis in weaned piglets
927 animals of about a month were used in the test. They were divided into 3 groups, namely a test group, where the product of the invention was administered for 14 days together with food beginning from the day of weaning, in a dose of 3 kg/t of food; a control group I, where piglets for 14 days did not get any preparation in the food; and a control group II, where piglets were administered a chemotherapeuticum in the prescribed dose for the same time period. Results are evident from Fig. 2,3 and 4. Fig. 2 shows the percentage of sickened piglets with signs of diarrhoea, Fig. 3 shows the percentage of dead piglets with signs of diarrhoea and Fig. 4 shows daily weight increase.
b) Activity in prevention of dysentery (bloody flux) in pigs
The test was carried out on a farm, where dysentery in clinical form appeared in fattening pigs 10 to 15 days before slaughter. 400 fattening pigs, weight about 100 kg, divided into two groups - the test group and the control group (200 animals in each group) - were tested. The test group was administered the product of the invention in a dose of 3 kg/t of food for 10 days and the control group was administered a chemotherapeuticum in the prescribed dose in the same time period. In the test group identical results of prevention as in the control group were achieved. In neither group clinical dysentery was observed in the last 20 days of fattening, though the disease appeared on the farm regularly in previous months.
c) Activity of the product of the invention if dysentery in pigs has already appeared
60 fattening pigs, weight 90 to 100 kg each, having clinical signs of dysentery were tested. They were divided into two groups - the test group and the control group. 30 fattening pigs, which were administered the product of the invention in the food (5 kg/t of food) for 7 days, were in the test group. 30 fattening pigs, which were administered a chemotherapeuticum in the prescribed dose in the same time period, were in the control group. The results are evident from the Table 3.

**TABLE 3**

| days | the number of pigs without dysentery | |
|---|---|---|
| | A | B |
| 1 | 0 (0 %) | 0 (0 %) |
| 2 | 5 (16.66%) | 0 (0 %) |
| 3 | 6 (20 %) | 6 (20 %) |
| 4 | 6 (20 %) | 6 (20 %) |
| 5 | 22 (73.33 %) | 6 (20 %) |
| 6 | 28 (93.33 %) | 18 (60 %) |
| 7 | 30 (100 %) | 30 (100 %) |
| A = the test group B = the control group | | |

4. Activity of the product of the invention against winter viral dysentery in cattle
69 adult animals in which winter viral dysentery had been established, were tested. The test lasted for 2 months. The product of the invention was administered individually as 2 x 4 big spoons of the solution (0.2 g of the product/kg body weight, dissolved in 2 to 3 l water, administered with a probe directly into the pre-gorge or by bottle) daily for 3 to 4 days. The faeces of the most animals treated became thicker and thicker on administration. At the end it was like a thick pulp or partly formed.

## Claims

1. Tannin extract of sweet chestnut wood comprising a pulverized solution of a) thickened aqueous extract of sweet chestnut wood (Castanea Sativa Mill.) without bark and sap-wood, **characterized** in that the tannin extract of sweet chestnut wood is derived from wood having an age from 40 to 50 years, and b) contains pharmaceutically acceptable sugar, the ratio dry substance of extract a)/sugar being from 1:2 to 1:6.

2. Process for preparing tannin extract of sweet chestnut wood for veterinary use, **characterized** in that the sound wood of sweet chestnut, of an age of 40 to 50 years, without bark and sap-wood, in the form of logs, which is ground into particles having a length of 6 to 10 mm, a thickness of 0.6 to 1.2 mm and a width of 4 to 8 mm, is extracted with water, the extraction solution is thickened to the dry substance content of about 50%, to the liquid extract a solution of microbiologically acceptable sugar is added in order to achieve a ratio dry substance of extract/sugar 1:2 to 1:6, it is stirred to complete homogeneity and pulverized.

3. Veterinary preparation active in the treatment of diarrhoea in animals, **characterized** in that it contains the tannin extract of claim 1 and conventional carriers and adjuvants.

4. Veterinary preparation according to claim 3, **characterized** in that it is included in animal fodder.

5. Veterinary preparation according to claim 3 for use in preventing and curing of diarrhoea, dysentery and other gastrointestinal disorders accompanied with diarrhoea in animals such as mammals and poultry.

## Patentansprüche

1. Tanninextrakt aus Edelkastanienholz enthaltend eine pulverisierte Lösung des a) eingedickten wässrigen Extraktes aus Edelkastanienholz (Castanea Sativa Mill.) ohne Rinde und Splintholz, dadurch gekennzeichnet, dass der Tanninextrakt aus 40 bis 50 Jahre altem Edelkastanienholz gewonnen wird und b) einen pharmazeutisch annehmbaren Zucker enthält, wobei das Verhältnis von Trockensubstanz des Extrakts a)/Zucker von 1:2 bis 1:6 beträgt.

2. Verfahren zur Herstellung von Tanninextrakt aus Edelkastanienholz für Verwendung in Veterinärmedizin, dadurch gekennzeichnet, dass gesundes, 40 bis 50 Jahre altes Edelkastanienholz ohne Rinde und Splintholz in der Form von Scheitholz, das zu 6 bis 10 mm langen, 0.6 bis 1.2 mm dicken und der 4 bis 8 mm breiten Teilchen zerkleinert wird, mit Wasser extrahiert wird, die Extraktionslösung bis zum Trockensubstanzgehalt von etwa 50% eingedickt wird, der flüssige Extrakt mit einer Lösung des mikrobiologisch annehmbaren Zuckers zur Erhaltung des Verhältnisses von Trockensubstanz des Extraktes/Zucker von 1:2 bis 1:6 versetzt wird, bis zur völligen Homogenität gerührt und pulverisiert wird.

3. Veterinärmedizinisches Präparat aktiv in der Behandlung von Diarrhöe bei Tieren, dadurch gekennzeichnet, dass es den Tanninextrakt gemäss Anspruch 1 sowie übliche Träger und Hilfsmittel enthält.

4. Veterinärmedizinisches Präparat gemäss Anspruch 3, dadurch gekennzeichnet, dass es im Tierfutter enthalten ist.

5. Veterinärmedizinisches Präparat gemäss Anspruch 3 zur Verwendung in der Präventive und Kurative von Diarrhöe, Dysenterie und anderen, mit Diarrhöe begleiteten gastrointestinalen Störungen bei Tieren wie Säugetieren und Geflügel.

## Revendications

1. Extrait de tanin de bois tendre de châtaignier, comprenant une solution pulvérisée de a) un extrait aqueux épaissi de bois tendre de châtaignier (*Castanea Sativa Mill.*) sans écorce et sans sève, caractérisé en ce que l'extrait de tanin de bois tendre de châtaignier provient d'un bois âgé de 40 à 50 ans, et b) il contient un sucre pharmaceutiquement acceptable, le rapport de la substance sèche d'extrait a) au sucre valant de 1:2 à 1:6.

2. Procédé de préparation d'un extrait de tanin de bois tendre de châtaignier pour un usage vétérinaire, caractérisé en ce qu'on extrait le bois sain de châtaignier tendre, âgé de 40 à 50 ans, sans écorce et sans sève, sous la forme de rondins, qui est broyé en particules d'une longueur de 6 à 10 mm, une épaisseur de 0,6 à 1,2 mm et une largeur de 4 à 8 mm, avec de l'eau, on épaissit la solution d'extraction jusqu'à ce que sa teneur en substances sèches soit d'environ 50 %, on ajoute à l'extrait liquide une solution de sucre pharmaceutiquement acceptable de façon à obtenir un rapport de la substance sèche d'extrait au sucre de 1:2 à 1:6, on l'agite jusqu'à homogénéité complète et on le pulvérise.

3. Préparation vétérinaire active dans le traitement des diarrhées chez les animaux, caractérisée en ce qu'elle contient l'extrait de tanin de la revendication 1, ainsi que des véhicules et des adjuvants usuels.

4. Préparation vétérinaire selon la revendication 3, caractérisée en ce qu'elle est contenue dans des aliments pour animaux.

5. Préparation vétérinaire selon la revendication 3, destinée à être utilisée pour empêcher ou guérir les diarrhées, la dysenterie et d'autres dysfonctionnements gastro-intestinaux accompagnés de diarrhées chez des animaux comme les mammifères et les volailles.
